Europäisches Patentamt

European Patent Office

Office européen des brevets

(11) Veröffentlichungsnummer: **0 052 288**
**B1**

(12) **EUROPÄISCHE PATENTSCHRIFT**

(45) Veröffentlichungstag der Patentschrift: **28.05.86**

(21) Anmeldenummer: **81109375.6**

(22) Anmeldetag: **30.10.81**

(51) Int. Cl.⁴: **A 23 J 3/00,** A 61 K 37/12,
A 61 L 15/01, A 61 L 2/18,
A 61 L 2/06, A 61 F 2/00,
C 07 K 1/00

(54) **Kollagenpräparate, Verfahren zu ihrer Herstellung und ihre Verwendung in der Human- und Veterinärmedizin.**

(30) Priorität: **13.11.80 DE 3042860**

(43) Veröffentlichungstag der Anmeldung:
**26.05.82 Patentblatt 82/21**

(45) Bekanntmachung des Hinweises auf die
Patenterteilung:
**28.05.86 Patentblatt 86/22**

(84) Benannte Vertragsstaaten:
**AT BE CH DE FR GB IT LI LU NL SE**

(56) Entgegenhaltungen:
**DE-B-1 063 330
US-A-3 093 439
US-A-3 455 776**

(73) Patentinhaber: **Heyl Chemisch-pharmazeutische
Fabrik GmbH & Co. KG
Goerzallee 253
D-1000 Berlin 37 (DE)**

(72) Erfinder: **Walter, Peter, Prof. Dr.
Waldstrasse 48
D-8034 Unterpfaffenhofen-Harthaus (DE)**
Erfinder: **Walter, Michael
Leopoldstrasse 97
D-8000 München 40 (DE)**

(74) Vertreter: **Kinzebach, Werner, Dr.
Patentanwälte Reitstötter, Kinzebach und
Partner Postfach 780
D-8000 München 43 (DE)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

## Beschreibung

Die Erfindung betrifft Kollagenpräparate, ein Verfahren zu ihrer Herstellung und ihre Verwendung in der Human- und Veterinärmedizin.

Die Häufigkeit und Bedeutung von Hautdefekten vor allem nach Verbrennungs- und anderen Unfallverletzungen sind bekannt. Zu ihrer Versorgung diente bisher neben der übrigen Wundbehandlung das autologe Hautpräparat. Seiner Verwendung sind jedoch von der Defektgröße her Grenzen gesetzt. Es hat deshalb nicht an Versuchen gefehkt Kollagene, die einen Hauptbestandteil von Lederhaut (Corium), Bindegewebe, Sehnen, Fascien und Bändern bilden, in der Medizin z.B als temporären Hautersatz, für Gefäßprothesen usw. anzuwenden (vgl. z.B. Umschau *66*, 1966, 230). Über bisherige Bemühungen, ein geeignetes Hautersatzmaterial zu entwickeln, gibt z.B. die Monographie "Experimental Skin Grafts and Transplantation Immunity" von D. L. Ballantyne und J. M. Converse, Springer-Verlag Berlin 1979 Auskunft. Für Blutgefäßprothesen werden seit einigen Jahren Modifizierte Blutgefäße vom Rind (sogenannte "Bovine heterograft") verwendet (vgl. z.B. Rosenberg, Surg. Forum *7*, 1965, 243). Solche Prothesen haben die Erwartungen jedoch noch nicht erfüllt, weil sich durch Übertritt von Stoffen aus dem durchströmenden Blut irreversible Wandveränderungen entwickeln. Sie werden daher heute kaum noch verwendet.

Die bisherige Verwendung von Kollagenpräparaten in der Medizin beschränkt sich auf Kollagenschwamm bzw. Kollagenvlies. Die biologische Textur des Kollagens ist in diesen Fällen zerstört, was zur Veränderung seiner physikalischen aber auch einiger biologischer Eigenschaften führt (vgl. z.B. M. Chvapil, Collagen Sponge: Theory and Practice of Medical Applications, J. Biomed. Mater. Res., *11*, 1977, 721 bis 741).

Aus der US—A—3 093 439 ist ein Verfahren zur Herstellung von Kollagenpräparaten bekannt, bei dem Kollagenmaterial tierischen Ursprungs unter anderem mit einer Ficin-Lösung und einer Dialdehyd-Stärkelösung behandelt wird.

Aufgabe der vorliegenden Erfindung ist es, modifizierte Kollagenpräparate tierischen Ursprungs für die Human- und Tiermedizin bereitzustellen, die längere Zeit gelagert und sofort trans- oder implantiert werden können, bei denen die erwünschten biologischen Eigenschaften (z.B. Keimabwehr, Anregung der Grundsubstanzproduktion von Zellen, Granulationsförderung, Blutstillung, usw.) des natürlichen Ausgangsmaterials erhalten geblieben sind und deren Form und physikalische Eigenschaften dem jeweiligen Verwendungszweck angepaßt sind.

Gegenstand der Erfindung sind daher Kollagenpräparate aus Kollagen Typ I für die Human- oder Tiermedizin, die dadurch erhältlich sind, daß man Säugetier-Kollagen-I-Material unter Bewahrung seiner biologischen Textur proteolysiert, mit Dialdehyden vernetzt und sterilisiert,

wobei man

A) die Proteolyse in Gegenwart von Ficin und L-Cystein in einem wässrigen Medium bei einem pH-Wert von 5,5 bis 6,5 und einer Temperatur von 30 bis 50°C durchführt,

B) die Vernetzung mit einem oder mehreren aliphatischen Dialdehyden mit 2 bis 10 Kohlenstoffatomen durchführt,

C) anschließend in wässrigem Medium reduziert und

D) gewünschtenfalls vor der Sterilisierung heiß behandelt, formt und/oder Stücke "verschweißt".

Als Säugetier-Kollagen-I-Material verwendet man Haut, Corium, Fascien, strangförmige Formationen aus Sehnen, röhrenförmige Formationen aus Blutgefäßen und schlauchförmigen Hohlorganen, sackförmige Formationen aus Herzbeuteln und interstitielles Mammagewebe.

Die vorstehend genannten Ausgangsmaterialien entnimmt man Säugetieren, vorzugsweise Wiederkäuern, insbesondere der Ziege, dem Rind und Rinderfeten.

Zur Vernetzung setzt man vorzugsweise Dialdehyde mit 4 bis 7 Kohlenstoffatomen, insbesondere Glutardialdehyd, vorzugsweise in 1 bis 10%iger wässriger Lösung ein.

Die Reduktion erfolgt mit Alkaliborhydrid, insbesondere Natriumborhydrid in wäßrigem Medium.

Die Heißbehandlung wird mit wasserdampffgesättigter Heißluft bei 80° bis 100°C einige Minuten durchgeführt.

Zur Formgebung und/oder "Verschweißung" von Präparatestücken wird das Präparat oder die mit den Rändern dicht aneinanderliegenden Präparatestücke auf einen die gewünschte Form aufweisenden inerten, festen Körper mit glatter Oberfläche aufgezogen und dort wird die Heißluftbehandlung fortgesetzt.

Wenn man von einem dicken Kollagenmaterial ausgeht, unterwirft man es zuerst einer ersten Proteolyse, bringt es dann durch Spaltung in Faserrichtung in die gewünschte Dicke, proteolysiert das so erhaltene Material erneut und behandelt es wie oben beschrieben weiter.

Vorzugsweise wird nach der Proteolyse eine Behandlung mit verdünnter wäßriger Natriumchlorit-lösung zwischengeschaltet.

Das Wässern zwischen den einzelnen Verfahrensstufen erfolgt mit fließendem Wasser und dauert int der Regel jeweils 24 Stunden.

Vorzugsweise behandelt man das Säugetier-Kollagen-I-Material 8 bis 24 Stunden mit einer Waßrigen Lösung, die 10 g Ficin/Liter und 1 g L-Cystein/Liter enthält bei einem pH-Wert von 6 und einer Temperatur von 37°C, behandelt dann 24 Stunden mit einer 1%-igen wäßrigen Natriumchloritlösung, wässert, behandelt 7 Tage mit einer 1 bis 10%-igen wäßrigen Glutaraldehydlösung, wässert, behandelt 24 Stunden mit einer waßrigen Natriumborhydridlösung, wässert und sterilisiert anschließend, wobei das Wässern mit fließen- dem Wasser erfolgt und jeweils 24 Stunden dauert.

Zwecks Gewinnung von Kollagenpräparaten bestehend aus schlauchförmigen Hohlorganen mit einem bestimmten Durchmesser zieht man ein formentsprechendes Kollagenmaterial nach der Reduktion und einer ersten kurzen Heißbehandlung auf einen stabförmigen, festen Körper mit glatter Oberfläche und dem gewünschten Durchmesser auf und behandelt mit wasserdampfgesättigter Heißluft von etwa 1,5 atü bei 80° bis 100°C einige Minuten lang weiter.

Als stabförmigen Körper verwendet man dabei vorzugsweise einen silikonbeschichteten Glasstab.

Zwecks Gewinnung eines Kollagenpräparats in Form eines beutelförmigen Hohlorgans mit bestimmtem Durchmesser zieht man nach der Reduktionsbehandlung und einer ersten kurzen Heißbehandlung den Herzbeutel eines Rindes auf einen silikonbeschichteten Glaskolben auf, setzt die Heißbehandlung fort, nimmt den geformten Beutel ab und sterilisiert in üblicher Weise.

Zwecks Gewinnung eines Kollagenpräparates in Form eines watte- oder wollartigen Füllmaterials geht man von geraspeltem Coriummaterial oder interstitiellem Mammagewebe aus.

Gegenstand der Erfindung ist auch ein Verfahren zur Herstellung der oben geschilderten Kollagen-I-Präparate, das die vorbezeichneten, zur Charakterisierung der Präparate aufgeführten, Verfahrensmaßnahmen beinhaltet.

Gegenstand der Erfindung ist außerdem die Verwendung der vorbezeichneten Präparate in der Human- und Tier-Medizin, für die chirurgische Versorgung von defekten Körperoberflächen, als Trans- oder Implantate zum Ersatz von Haut, Bändern, Sehnen, Sehnenscheiden, Hohlorganen, Blutgefäßen und der weiblichen Brust.

Als Ausgangsmaterial für die erfindungsgemäßen Präparate wird Kollagen-I-material, vorzugsweise von der Ziege, insbesondere vom Rind und Rinderfeten, verwendet, das aus mindestens etwa 90% Kollagen vom Typ I besteht, das sind z.B die Haut, das Corium, Sehnen, Bänder, Blutgefäße, der Herzbeutel und das interstizielle Mammagewebe der genannten Säugetiere. Die Art des Ausgangsmaterials richtet sich dabei insbesondere nach dem beabsichtigten Verwendungszweck in der Human- oder Tiermedizin:

Zur Vesorgung von Körperoberflächen - (Haut) - defekten (heterologes Hauttransplantat, Wundpflaster, Tupfer), z.B. nach Verbrennungen und anderen Unfallverletzungen, werden Häute in ihrer biologischen Textur eingesetzt.

Zur Förderung der Osteoneogenese nach Frakturen mit und ohne Substanzverlust werden Fascien unterschiedlicher Stärke und vor allem das Corium (Lederhaut) insbesondere von erwachsenen Rindern, ohne Epidermisdecke, als solches oder auch in dünne Schichten gespalten, verwendet.

Für Bandersatzpräparate (heterologer Bandersatz bei Rupturen von Gewebsbändern) werden strangförmige Formationen aus Sehnen, wie z.B. Biegesehnen von Rindern verwendet.

Zur Herstellung elastischer Schläuche für defekte schlauchförmige Hohlorgane (Blutgefäße, Gallengang, usw.) dienen vorzugsweise röhrenförmige Formationen aus schlauchförmigen Hohlorganen, wie aus Arterien gewonnene Kollagenschläuche, und zum Schlauch vernähte Fascien, während für den Sehnenscheidenersatz praktisch alle Kollagenformationen, wie die oben genannten, verwendet werden können.

Zur Herstellung sackförmiger Gebilde, z.B. Ersatzpräparate für die weibliche Brust, geht man vom Herzbeutel des Rindes aus. Zur Gewinnung von watte- oder wollartigem Füllmaterial geht man von geraspeltem Material, insbesondere geraspeltem Coriummaterial, oder von interstiziellem Mammagewebe aus.

Dicke Kollagensysteme, wie z.B. strangförmige Formationen aus Sehnen, werden vorzugsweise vor dem eigentlichen Verfahren einer Proteolyse unterworfen und dann parallel zur Faserrichtung durch Längsspaltung in die gewünschte Dicke gebracht. So wird z.B. der etwa mittelfingerstarke und 30 bis 40 cm lange verwendbare Teil der Beugesehnen von Rindern vorzugsweise zunächst als Ganzes einer 24-stündigen Proteolyse unterworfen, dann parallel zur Faserrichtung durch Längsspaltung das gewünschte Bandkaliber (z.B. mit einer Stärke von ca. 2,5 mm und darüber) gewonnen, woran sich einer nachfolgende 24-stündige Proteolyse anschließt. Ein Endes des Bandes wird danach vorzugsweise geknotet und dann den weiteren Verfahrensschritten unterworfen.

Werden behaarte Häute als Ausgangsmaterial verwendet, so erfolgt vor dem eigentlichen Verfahren eine Enthaarung, wobei man zweckmäßigerweise nach dem aus der Gerberei-Technik bekannten "Äschern" arbeitet. Vorzugsweise werden dazu die Häute in gelöschten Kalk eingelegt, dem pro kg 1 g Natriumsulfit zugesetzt wird. Nach einer Verweildauer von 6 bis 10 Tagen werden die Häute gewässert, anschließend für 3 Stunden in 1%-ige Essigsäure eingelegt und dann nochmals 24 Stunden gewässert.

Zweckmäßigerweise sollte das Ausgangsmaterial unmittelbar nach der Tötung (Schlachtung) der Tiere gewonnen werden. Wenn sich daran nicht sofort das erfindungsgemäße Präparationsverfahren anschließt, so ist eine Konservierung der Kollagensysteme durch Einfrieren und Lagerung bei Tiefkühltemperaturen nahezu praktisch unbegrenzt möglich. Eine solche Konservierung ist auch noch nach einer Enthaarung möglich.

Das erfindungsgemäße Verfahren ist für alle genannten Ausgangsmaterialien gleichermaßen gut geeignet. Es kann aber zweckmäßig sein, je nach der Art des eingesetzten Ausgangsmaterials in einer oder mehreren der Verfahrensstufen verschiedene Reaktionszeiten, Reaktionstemperaturen und/oder Konzentrationen einzu halten.

Die Proteolyse führtman in Gegenwart von Ficin und L-Cystein durch, wobei man in einer wäßrigen Lösung bei einem pH-Wert von ca. 5,5 bis ca. 6,5 und insbesondere 6, und bei 30 bis 50°C, insbesondere etwa 37°C arbeitet. Die Einstellung des pH-Wertes erfolgt dabei zweckmäßigerweise durch Zusatz von 1N Trinatriumcitratpuffer. Die Reaktionszeit beträgt, abhängig von der Dicke der Kollagensysteme, im allgemeinen 8 bis 24 Stunden.

Zur Vernetzung der Peptidstrukturen der Kollagen-I-Präparate verwendet man Alkandiale mit 2 bis 10 und insbesondere 4 bis 7 Kohlenstoffatomen. Als besonders zweckmäßig hat sich dabei aufgrund seines hohen Penetrationsvermögens und dem hohen Diffusionsgrad Glutardialdehyd erwiesen. Die Modifizierung des Vernetzungsgrades des Kollagens und damit die Steifigkeit des Materials ist stark von der Konzentration des Vernetzungsmittels abhängig. Insbesondere die Herstellung von Material für Hohlorgane (Blutgefäße usw) erfordert ein besonders weiches, flexibles Material. Die Konzentration (Angaben beziehen sich auf Glutardialdehyd) liegen im allgemeinen zwischen 1 und 10 Gew.-%, wobei die bevorzugten Konzentrationen bei Häuten mit biologischer Textur, Fascien und Corium 3 bis 5%, für strangförmige Formationen aus Sehnen 5 bis 10%, für röhrenförmige Formationen aus schlauchförmigen Hohlorganen 1 bis 3% und für die Herstellung eines heterologen Sehnenscheidenersatzes bei 5 bis 8% liegt. Im allgemeinen wird das Material in eine Wäßrige Lösung des Aldehyds eingebracht und darin mehrere Tage, zweckmäßigerweise ca. 7 Tage, bei Raumtemperatur belassen.

Die Reduktion wird mit einem in der Lederindustrie verwendeten reduzierenden Bleichmittel unter den dafür üblichen Bedingungen durchgeführt, insbesondere aber mit einem Alkaliborhydrid, wie z.B. Natriumborhydrid. Durch diese Reduktion wird Unlöslichkeit des Kollagens in saurem und alkalischem Milieu erreicht und gleichzeitig die durch den Quervernetzungsprozeß eingetretene Starre des Materials weitgehend aufgehoben. Das Material wird geschmeidiger, behält aber seine Reißfestigkeit. Der damit außerdem verbundene zusätzliche Bleicheffekt ist, insbesondere für die Anwendung als Wundpflaster, sehr vorteilhaft. Die Reduktion mit Alkaliborhydrid erfolgt in wäßrigem Medium. Man kann dabei im allgemeinen soviel Natriumborhydrid anwenden, wie dies zur Erreichung einer gleichmäßig weiß gebleichten Oberfläche erforderlich ist; in der Regel arbeitet man mit 0,5 bis 1,5 g Natriumborhydrid/Liter Wasser. Zur Reduktion wird das Material in die wäßrige Natriumborhydridlösung eingebracht und darin mehrere Stunden, in der Regel 4 bis 24 Stunden, bei Raumtemperatur belassen.

Die Sterilisation kann nach einer der zur Sterilisation von chirurgischem Material verwendeten üblichen Methoden durchgeführt werden; als besonders geeignet, insbesondere auch für eine nachfolgende gebrauchsfähige Lagerung, hat sich eine Sterilisation mit wäßrigem Alkohol in Gegenwart geringer Mengen an Propylenoxid, wie z.B. Einlagern des Materials in 50%-iges wäßriges Äthanol, das 1 Gew.-% Propylenoxid enthält, während ca. 10 Tagen erwiesen. Das auf diese Weise sterilisierte Material wird anschließend in eine sterile, gepufferte Kochsalzlösung (BSS) eingelegt, aus der man den Rest der Sterilisationslösung abdampft (z.B. im Thermostat während 3 bis 8 Stunden). In diesem Zustand ist das Material lagerfähig und gebrauchsfertig. Zur Vermeidung einer Ablagerung von Calciumsalzen soll die Kochsalzlösung frei sein von Calciumionen. Wird im Hinblick auf die spätere Anwendung eine Lagerung in einer sterilen Vakuumverpackung, also in trockener Form, oder in einer nativen Kollagenlösung bevorzugt, so kann sich an die Sterilisation eine Lyophilisation (Gefriertrocknung) oder auch eine Trocknung an der Luft anschließen. Insbesondere bei Hautersatzpräparaten ist eine Aufbewahrung in einer nativen Kollagenlösung zweckmäßig, weil sie ihrerseits den Heilprozeß güstig beeinflußt.

Um zu verhindern, daß trotz Wässerung die Proteolyse durch nicht vollständig entfernte Proteasereste in geringem Umfang weitergeht, ist es sehr zweckmäßig, nach dem Verfahrensschritt der Proteolyse eine Behandlung mit Natriumchlorit (NaClO$_2$) zwischenzuschalten, wodurch die Proteolyse gestoppt wird. Vorzugsweise wird dazu eine verdünnte wäßrige Natriumchloritlösung, z.B. eine 1%-ige Lösung, verwendet.

Das nach dem Verfahrensschritt der Reduktion erhaltene Kollagenmaterial kann vor der Sterilisation zur Verbesserung der Elastizität einer Hitzebehandlung in mit Wasserdampf gesättigter Luft unterworfen werden, wobei man in der Regel einige Minuten bei 80 bis maximal 100°C und einem Druck von 1,5 atü arbeitet. Die Hitzebehandlung kann in einem üblichen Dampfsterilisationsgefäß durchgeführt werden.

Zur Erzeugung glatter, geschlossener Oberflächen kann das Kollagenmaterial in einem zweiten Teil dieser Hitzebehandlung mit der Oberfläche eines gegen das Material inerten, glatten, festen Gegenstandes, wie z.B. Glas, in Kontakt gebracht werden, der auf ca. 80 bis maximal 100°C aufgeheizt ist. Gut geeignet sind dafür auch silikonbeschichtete Oberflächen. Diese Heißkontaktierung dauert in der Regel einige Minuten, z.B. 1 bis 15 Minuten.

Die Heißkontaktierung ist insbesondere für die Abdichtung der inneren Oberflächen von Hohlorganen, wie z.B. von Blutgefäßen, von großer Bedeutung, da auf diese Weise völlig dichte und glatte innere Oberflächen erzielt werden, im Unterschied zu den bisher verwendeten modifizierten Blutgefäßen ("Bovine heterograft") nach dem Stand der Technik.

Nach diesem Verfahren lassen sich Gefäße mit einem bestimmten Durchmesser relativ leicht dadurch herstellen, daß man das Kollagenmaterial (z.B. aus Arterien gewonnene Schläuche oder zum Schlauch vernähte Fascienpräparate) auf einen stabförmigen Träger, z.B. einen silikonbeschichteten Glasstab vorgegebener Stärke aufzieht und danach der Heißbehandlung unterwirft. Dabei schrumpft das Ausgangsmaterial auf dem Glasstab und erhält auf seiner ganzen Länge gleichmäßig den vorgegebenen Durchmesser. Auf diese Weise können durch geeignete Wahl des Durchmessers des Trägermaterials die Durchmesser der Gefäße eingestellt werden. Es wird außerdem die Elastizität von Gummischläuchen bei absoluter Dichter erreicht.

Auf die gleiche Weise können auch Materialstücke ohne Vernähung oder andere mechanische Hilfsmittel durch Heißbehandlung völlig dicht verschweißt werden, wobei die Stoßstellen gewissermaßen miteinander verschmelzen. Dies ist insbesondere sehr wichtig bei der Verbindung von Gefäßstücken, da die natürlichen Quellen, nämlich die isolierten Gefäßstücke, nur eine begrenzte Länge haben und bisher längere Gefäßstücke nur durch Aneinandernähen von kürzeren Stücken erhältlich waren. Dieser Vernähungsprozeß kann durch den Verschweißungsprozeß ersetzt werden, was insbesondere den Vorteil hat, daß an den ,Verschweißungsstellen keine Undichtigkeiten auftreten.

Die nach dem erfindungsgemäßen Verfahren erhaltenen Kollagenpräparate eignen sich hervorragend für die chirurgische Versorgung von defekten Köperoberflächen, von Frakturen, Rupturen, defekten Sehnenscheiden und defekten Hohlorganen. Gegenstand der Erfindung ist deshalb auch die Verwendung der nach dem erfindungsgemäßen Verfahren erhaltenen Kollagenpräparate.

Die erfindungsgemäß hergestellten Präparate werden vom Empfänger nicht nur toleriert, sondern integriert. Körperflächenabdeckungen werden kurzfristig integriert und nach erfolgter Granulation abgestoßen. Es hat sich gezeigt, daß der von Rindern und Ziegen erhaltene Kollagentyp für den Menschen unbedenklich ist, weil seine Aminosäuresequenz weitgehend derjenigen des menschlichen Kollagens entspricht.

Beispiel 1

Das Fell eines Rindes oder einer Ziege wirdwie in der Gerberei üblich—enthaart, indem man es in gelöschten Kalk, der 1 g Natriumsulfit pro kg gelöschtem Kalk enthält, einlegt. Nach 6 bis 10 Tagen wird die Tierhaut gewässert, anschließend 3 Stunden in 1%-ige Essigsäure eingelegt und dann nochmals 24 Stunden gewässert. Die erhaltene Haut ist enthaart.

Aus dieser Haut schneidet man ein Stück von 40×40 cm und legt dieses Hautstück in 3 Liter Proteolyselösung, die mit 1 N Trinatriumcitrat-Puffer auf pH 6 eingestellt worden ist. Die 3 Liter Proteolyselösung enthalten 30 g Ficin und 3 g L-Cystein. Man läßt die Proteolyselösung etwa 16 Stunden lang bei 37°C auf das Hautstück einwirken. Anschließend wird 24 Stunden unter fließendem Wasser gewässert. Dann wird das Hautstück 24 Stunden in eine 1 %-ige Natriumchlorit-Lösung (NaClO$_2$) anschließend wieder 24 Stunden in fließendes Wasser gelegt. Danach legt man das Hautstück 7 Tage in eine wäßrige, 3%-ige Glutardialdehydlösung, in der es von Zeit zu Zeit bewegt wird, legt anschließend wieder 24 Stunden in fließendes Wasser, überführt das Präparat dann in eine wäßrige, 1%-ige Natriumborhydrid (NaBH$_4$) Lösung, wässert erneut 24 Stunden, sterilisiert 10 Tage lang bei Raumtemperatur in einer wäßrigen, 50%-igen Äthylalkohollösung, die 1% Propylenoxyd enthält, legt dann das Präparat in eine sterile, gepufferte Kochsalzlösung, heizt die Lösung in Thermostaten auf 60 bis 90°C und verdampft 3 bis 8 Stunden lang den Rest der Sterilisationslösung. Das so erhaltene fertige Präparat kann man entweder in nativer Kollagenlösung afbewahren oder lyophilisieren und in steriler Vakuumverpackung lagern.

Beispiel 2

Acht Beugesehnen von Rindern (30 bis 40 cm lang, mit einem Durchmesser von 2 bis 2,5 cm) werden 24 Stunden bei pH 6 und 37°C in 3 Liter der in Beispiel 1 beschriebenen Proteolyselösung liegengelassen. Dann spaltet man die Sehnen längs, parallel zum Faserverlauf in etwa 2 bis 3 mm starke Bänder und legt die erhaltenen Bänder nochmals 24 Stunden lang bei 37°C und pH 6 in 3 Liter der in Beispiel 1 beschriebenen Proteolyselösung. Man wässert und behandelt mit Natriumchloritlösung, wie in Beispiel 1 beschrieben, und verwendet zur Vernetzung eine 4%-ige, wäßrige Glutardialdehydlösung. Die Weiterbehandlung und Sterilisierung erfolgt wie in Beispiel 1.

Beispiel 3

Rinderhaut wird, wie in Beispiel 1 beschrieben, enthaart. Dann entfernt man in an sich bekannter Weise die Epidermis, um das Corium zu gewinnen. Ein Stück Corium von etwa 40×40 cm wird eingefroren und anschließend durch Raspeln zerfasert. Das geraspelte Material wird in 3 Liter Proteolyselösung, wie in Beispiel 1 beschrieben, proteolysiert, auch die Weiterbehandlung erfolgt, wie in Beispiel 1 beschrieben. Man erhält ein fasriges, wolliges Material.

Beispiel 4

Aus dem Euter einer frisch geschlachteten Kuh werden 800 g interstizielles Mammagewebe isoliert, und gemäß den Angaben des Beispiels 1 proteolysiert und weiterbehandelt. Man erhält, ähnlich wie in Beispiel 3, ein fasriges, wolleartiges Füllmaterial.

Beispiel 5

8 bis 10 Körperarterienstücke von 30 bis 50 cm Länge und 1 bis 2,5 cm Durchmesser, isoliert aus frisch geschlachteten Rindern, gibt man in 3 Liter Proteolyselösung, wie in Beispiel 1 beschrieben und behandelt diese Präparate nach den Angaben des Beispieles 1. Zur Vernetzung verwendet man allerdings eine 1 bis 2%-ige wäßrige Glutardialdehydlösung und läßt diese etwa 12 bis 16 Stunden lang einwirken. Die weiteren Schritte bis einschließlich der Reduktion erfolgen nach den Angaben des Beispiels 1. Danach unterwirft man die erhaltenen Arterienstücke in einem üblichen Dampfsterilisationsgefäß einer Heißbehandlung mit wasserdampfgesättigter Luft bei etwa 80 bis maximal 100°C und einem Druck von etwa 1,5 atü. Die Behandlungsdauer beträgt etwa 2 bis 8 Minuten, Man bereitet außerdem einen oder mehrere Glasstäbe vor, die mit einem dünnen Silikonfilm überzogen sind und zieht die der Heißbehandlung entnommenen Artierienstücke auf den oder die Glasstäbe auf. Man kann mehrere Arterienstücke hintereinander auf einen Glasstab aufziehen, so daß die Ränder der Arterienstücke dicht aneinanderstoßen. Dann unterwirft man das Ganze nochmals für 2 bis 10 Minuten der zuvor geschilderten Heißbehandlung im Dampfsterilisator. Dabei "verschmelzen" die aneinanderstoßenden Ränder der Arterienstücke und alle Arterienstücke nehmen den vom Glasstab vorgegebenen Innendurchmesser an. Nach einigen Minuten entnimmt man das Präparat dem Sterilisator und zieht den Glasstab aus dem Präparat heraus. Man erhält auf diese Weise einen Kollagenschlauch von gleichmäßigem Innendurchmesser und mit völlig glatter und dichter Innenfläche. Die Sterilisierung und Aufbewahrung kann in der in Beispiel 1 beschriebenen Weise erfolgen.

Beispiel 6

Den Herzbeutel einer frisch geschlachteten Ziege unterwirft man der in Beispiel 1 beschriebenen Proteolysebehandlung und den weiteren in Beispiel 1 beschriebenen Verfahrensschritten bis einschließlich der sich an die Reduktion anschließenden Wässerung. Für die Vernetzung verwendet man allerdings eine 1%-ige Glutardialdehydlösung, die man etwa 12 bis 16 Stunden lang einwirken läßt. Nach der Reduktion und Wässerung wird der Herzbeutel in einem Dampfsterilisator bei 90°C und etwa 1,5 atü etwa 5 Minuten der im vorhergehenden Beispiel beschriebenen Heißbehandlung unterworfen. Dann wird der der Heißbehandlung entnommene Herzbeutel auf die Siliconfilm-beschichtete konvexe Seite der Glasschale aufgezogen und für weitere 5 bis 10 Minuten in den Dampfsterilisator zurückgegeben. Man entnimmt das Präparat dem Sterilisator, läßt abkühlen, zieht das Präparat von der Glasschale ab und sterilisiert, wie in Beispiel 1 beschrieben.

Anwendungsbeispiele

A) Ein gemäß Beispiel 4 hergestelltes, der weiblichen Brust nachgebildetes Kollagenpräparat, wird mit einem gemäß Beispiel 3 bzw. 4 hergestellten Füllmaterial gefüllt und in der plastischen Chirurgie als Brustersatz verwendet.

Das gemäß den Beispielen 3 bzw. 4 hergestellte Füllmaterial kann in der plastischen Chirurgie auch zur Brustvergrößerung verwendet werden. Gegeüber den bisher für diese Zwecke verwendeten Silikonmaterialien hat es entscheidende Vorteile. Der erfindungsgemäße Füllstoff wandert nicht, sondern wird allmählich voll in das natürliche Brustgewebe integriert.

Aus einem gemäß Beispiel 2 hergestellten Coriumpräparat werden Wundpflaster, Tupfer und Tampons zugeschnitten. Nach dem Lyophilisieren werden sie steril und trocken verpackt. Sie können dann jederzeit als Pflaster, Tupfer und Tampons verwendet werden, die auch in der Wunde verbleiben können.

C) Ein gemäß Beispiel 1 hergestelltes Präparat wird am Operationstisch vor der Anwendung mit der Schere in die benötigte Form und Größe gebracht. Man legt das flächige Präparat auf die Wunde, näht es mit fortlaufender Naht fest, wobei exakt auf Stoß genäht wird. Die Fäden werden nach 14 Tagen gezogen. Die Abstoßung des Implantats beginnt mit einem Abheben der Ränder nach 18 bis 20 Tagen und ist nach etwa 3 bis 7 Wochen beendet.

D) Ein gemäß Beispiel 5 hergestelltes schlauchförmiges Präparat wird bei einer Sehnenruptur, vor dem Zusammennähen der Sehne über den Sehnenstumpf gestülpt und nach dem Vernähen der Sehne in die endgültige Position gebracht. Man kann das schlauchförmige Präparat auch vor der Applikation längs spalten, um die vernähte Sehne legen und anschließend mit evertierter Naht dicht vernähen. Man erhält auf diese Weise einen vorzüglichen Sehnenscheidenersatz, der in das Körpergewebe integriert wird.

**Patentansprüche**

1. Kollagenpräparate aus Kollagen I für die Human- oder Teirmedizin, dadurch erhältlich, daß Säugetier - Kollagen - I - Material unter Bewahrung seiner biologischen Textur proteolysiert, mit Dialdehyden vernetzt und sterilisiert, dadurch gekennzeichnet, daß man

A) die Proteolyse in Gegenwart von Ficin und L-Cystein in einem Wässrigen Medium bei einem pH-Wert von 5,5 bis 6,5 und einer Temperatur von 30 bis 50°C durchführt,

B) die Vernetzung mit einem oder mehreren aliphatischen Dialdehyden mit 2 bis 10 Kohlenstoffatomen durchführt,

C) anschließend in wässrigem Medium reduziert und

D) gewünschtenfalls vor der Sterilisierung heiß behandelt, formt und/oder Stücke "verschweißt".

2. Kollagenpräparate nach Anspruch 1, dadurch erhältlich, daß man zur Vernetzung Dialdehyde mit 4 bis 7 Kohlenstoffatomen insbesondere Glutardialdehyd, vorzugsweise in 1—10 %iger wässriger Lösung, verwendet, die Reduktion im wässrigen Medium mit Alkaliborhydrid durchführt und die Heißbehandlung mit wasserdampfgesättigter Heißluft bei 80° bis 100°C einige Minuten durchfuhrt und zur Formgebung und/oder "Verschweißung" von Präparatestücken das Präparat oder die mit den Rändern dicht aneinanderliegenden Präparatestücke auf einen die gewünschte Form aufweisenden inerten, festen Körper mit glatter Oberfläche aufzieht und dort die Heißbehandlung einige Minuten fortsetzt und dann sterilisiert.

3. Kollagenpräparate nach Anspruch 1, dadurch erhältlich, daß man als Säugetier - Kollagen - I - Material Haut, Corium, Fascien, Sehnen, Blutgefäße, schlauchförmige Hohlorgane, Herzbeutel und interstitielles Mammagewebe verwendet und daß man Kollagen - I - Material von Ziegen, Rindern und Rinderfeten einsetzt.

4. Kollagenpräparate nach einem der Ansprüche 1 bis 3, dadurch erhältlich, daß man dickes Kollagen-Material einer ersten Proteolyse unterwirft, dann durch Spaltung in Faserrichtung in die gewünschte Dicke bringt, das so erhaltene Material erneut proteolysiert und gemäß Anspruch 1 oder 2 weiterbehandelt.

5. Kollagenpräparate nach einem der Ansprüche, 1, 2 oder 4, dadurch erhältlich, daß man nach der Proteolyse eine Behandlung mit verdünnter wässriger Natriumchloritlösung zwischenschaltet und gegebenenfalls zwischen den einzelnen Verfahrensstufen mit fließendem Wasser wässert.

6. Kollagenpräparate nach einem der Ansprüche 1 bis 5, dadurch erhältlich, daß man das Säugetier - Kollagen - I - Material 8 bis 24 Stunden mit einer wässrigen Lösung, die 10 g Ficin/Liter und 1 g L-Cystein/Liter enthält, bei einem pH-Wert von 6 und einer Temperatur von etwa 37°C behandelt, dann 24 Stunden mit einer 1%-igen wässrigen Natriumchloritlösung behandelt wässert, 7 Tage mit einer 1 bis 10%-igen wässrigen Glutardialdehydlösung behandelt, wässert, 24 Stunden mit einer wässrigen Natriumborhydridlösung behandelt, wässert und anschließend sterilisiert, wobei das Wässern nit fließendem Wasser. erfolgt und jeweils 24 Stunden dauert.

7. Kollagenpräparate nach einem der Ansprüche 1 bis 6, dadurch erhältlich, daß man zwecks Gewinnung schlauchförmiger Hohlorgane mit einem bestimmten Durchmesser ein formentsprechendes Kollagenmaterial nach der Reduktion und einer ersten kurzen Heißbehandlung auf einen stabförmigen, festen Körper mit glatter Oberfläche und dem gewünschten Durchmesser, vorzugsweise eine silikonbeschichteten Glasstab, aufzieht, mit wasserdampfgesättigter Heißluft von etwa 1,5 atü bei 80° bis 100°C einige Minuten Behandelt und sterilisiert.

8. Kollagenpräparate nach einem der Ansprüche 1 bis 6, dadurch erhältlich, daß man zwecks Gewinnung eines beutelförmigen Hohlorgans mit bestimmten Durchmesser den Herzbeutel eines Rindes nach der Reduktionsbehandlung und einer ersten kurzen Heißbehandlung auf einen silikonbeschichteten Glaskolben mit dem gewünschten Durchmesser aufzieht, die Heißbehandlung kurz fortsetzt, den geformten Beutel abnimmt und sterilisiert.

9. Kollagenpräparate nach einem der Ansprüche 1 bis 6, dadurch erhältlich, daß man zwecks Gewinnung eines watte- oder wollartigen Füllmaterials von geraspeltem Coriummaterial oder interstitiellen Mammagewebe ausgeht.

10. Kollagenpräparate nach Anspruch 1, 2, 4, 6, 7 oder 12, dadurch erhältlich, daß man zum Zwecke des Sterilisierens das fertige Kollagenpräparat einige Tage lang in etwa 50%-igem wässrigen Ethylalkohol, der etwa 1% Propylenoxid enthält, einlegt, dann die Präparate in eine sterile, gepufferte Kochsalzlösung überführt, die Kochsalzlösung erwärmt, so daß der Rest der Sterilisationslösung im Verlaufe von mehreren Stunden verdampft.

11. Verwendung der Kollagenpräparate nach einem der Ansprüche 1 bis 10 in der Human- und Tiermedizin für die chirurgische Versorgung von defekten Körperteilen, als Trans- oder Implantate zum Ersatz von Haut, Bändern, Sehnen, Sehnenscheiden, Hohlorganen, Blutgefäßen und der weiblichen Brust.

12. Verfahren zur Herstellung der Kollagenpräparate nach den Ansprüchen 1 bis 10, wobei man Säugetier - Kollagen - I - Material unter Bewahrung seiner biologischen Textur proteolysiert, mit Dialdehyden vernetzt und sterilisiert, dadurch gekennzeichnet, daß man

A) die Proteolyse in Gegenwart von Ficin und L-Cystein in einem wässrigen Medium bei einem pH-Wert von 5,5 bis 6,5 und einer Temperatur von 30 bis 50°C durchführt,

B) die Vernetzung mit einem oder mehreren aliphatischen Dialdehyden mit 2 bis 10 Kohlenstoffatomen durchführt,

C) anschließend in wässrigem Medium reduziert und

D) gewünschtenfalls vor der Sterilisierung heiß behandelt, formt und/oder Stücke "verschweißt".

## Revendications

1. Préparation de collagène à partir de collagène I pour la médecine humaine ou vétérinaire, que l'on obtient en soumettant à la protéolyse du matériel de collagène I de mammifères, tout en préservant sa texture biologique, en le réticulant avec des dialdéhydes, et en le stérilisant, caractérisée en ce que

A) on effectue la protéolyse en présence de ficine et de L-cystéine, dans un milieu aqueux, à une valeur de pH allant de 5,5 à 6,5 et à une température allant de 30 à 50°C,

B) on effectue la réticulation avec un ou plusieurs dialdéhydes aliphatiques ayant de 2 à 10 atomes de carbone,

C) on réduit ensuite en milieu aqueux, et

D) si on le désire, on traite à chaud avant la stérilisation, on met en forme et/ou on "soude" les pièces.

2. Préparation de collagène selon la revendication 1, que l'on obtient en utilisant pour la réticulations des dialdéhydes ayant de 4 à 7 atomes de carbone, en particulier le dialdéhyde glutarique, de préférence en solution aqueuse à 1—10%, en effectuant la réduction en milieu aqueux avec un borohydrure alcalin, et en effectuant le traitement à chaud avec de l'air chaud saturé en vapeur d'eau à une température allant de 80 à 100°C pendant quelques minutes, et, pour la mise en forme et/ou la "soudure" des pièces de préparation, en étirant la préparation, ou les pièces de préparation dont les bords sont en contact étanche, sur un corps solide inerte, présentant la forme désirée et une surface unie, et en effectuant le traitement à chaud pendant quelques minutes, et en stérilisant ensuite.

3. Préparation de collagène selon la revendication 1, que l'on obtient en utilisant comme matériau de collagène I de mammifères, la peau, le corium, les fascias, les tendons, les vaisseaux sanguins, les organes creux en forme de tuyaux souples, le péricarde et le tissu mammaire interstitiel, et en utilisant du matériau de collagène I provenant de chèvre, de boeuf, et de foetus de boeuf.

4. Préparation de collagène selon l'une des revendications 1 à 3, que l'on obtient en soumettant à une première protéolyse un matériau de collagène épais, en l'amenant ensuite à l'épaisseur désirée par fendage dans le sens des fibres, en soumettant le matériau ainsi obtenu à une nouvelle protéolyse, et en le traitant ensuite selon la revendication 1 ou 2.

5. Préparation de collagène selon l'une des revendications 1, 2 ou 4, que l'on obtient en intercalant après la protéolyse un traitement avec une solution aqueuse diluée de chlorite de sodium, et en effectuant éventuellement des lavages à l'eau courante entre les diverses étapes du procédé.

6. Préparation de collagène selon l'une des revendications 1 à 5, que l'on obtient en traitant le matériau de collagène I de mammifère pendant 8 à 24 heures avec une solution aqueuse qui contient 10 g/l de ficine et 1 g/l de L-cystéine, à une valeur de pH de 6 et à une température d'environ 37°C, en le traitant ensuite pendant 24 heures avec une solution aqueuse à 1% de chlorite de sodium, en le lavant, en le traitant pendant 7 jours avec une solution aqueuse à 1 à 10% de dialdéhyde glutarique, en le lavant, en le traitant pendant 24 heures avec une solution aqueuse de borohydrure de sodium, en le lavant et enfin en le stérilisant, les lavages étant effectués avec de l'eau courante et durant chacun 24 heures.

7. Préparation de collagène selon l'une des revendications 1 à 6, que l'on obtient en enfilant, dans le but d'obtenir des organes creux en forme de tuyau souple ayant un diamètre déterminé, un matériau de collagène de forme correspondante, après la réduction et un premier traitement à chaud de courte durée, sur un corps solide en forme de barreau, présentant une surface unie et le diamètre désiré, de préférence un barreau de verre recouvert de silicone, en le traitant pendant quelques minutes avec de l'air chaud saturé en vapeur d'eau, sous une pression d'environ 1,5 bar à une température de 80 à 100°C, et en le stérilisant.

8. Préparation de collagène selon l'une des revendications 1 à 6, que l'on obtient en tendant, dans le but d'obtenir un organe creux en forme de sac ayant un diamètre déterminé, le péricarde d'un boeuf, après le traitement de réduction et un premier traitement à chaud de courte durée, sur un ballon de verre recouvert de silicone ayant le diamètre désiré, en effectuant un traitement à chaud de courte durée, en retirant le sac formé, et en le stérilisant.

9. Préparation de collagène selon l'une des revendications 1 à 6, que l'on obtient en partant, dans le but d'obtenir un matériau de remplissage ayant l'aspect de la ouate ou de la laine, d'un matériau de corium rapé ou de tissu mammaire interstitiel.

10. Préparation de collagène selon la revendication 1, 2, 4, 6, 7 ou 12, que l'on obtient en introduisant, dans le but de la stériliser, la préparation de collagène finie pendant quelques jours dans de l'alcool éthylique aqueux à environ 50%, contenant environ 1% d'oxyde de propylène, puis en faisant passer la préparation dans une solution de sel de cuisine stérilisé et tamponné, en chauffant la solution de sel de cuisine de telle façon que le résidu de la solution de stérilisation s'évapore en l'espace de plusieurs heures.

11. Utilisation d'une préparation de collagène selon l'une des revendications 1 à 10 en médecine humanine et vétérinaire, pour les soins chirurgicaux de parties du corps défectueuses, en tant que transplant ou implant de remplacement de la peau, des ligaments, des tendons, de gaines tendineuses, des organes creux, des vaisseaux sanguins et des seins.

12. Procédé de préparation des préparations de collagène selon les revendications 1 à 10, dans lequel on soument à une protéolyse un matériau de collagène I de mammifène, tout en conservant sa texture biologique, on le réticule avec des dialdéhydes, et on le stérilise, caractérisé en ce que

A) on effectue la protéolyse en présence de ficine et de L-cystéine, en milieu aqueux, à une valeur de pH allant de 5,5 à 6,5 et une température allant de 30 à 50°C,

B) on effectue la réticulation avec un ou plusieurs dialdéhydes aliphatiques ayant de 1 à 10 atomes de carbone,

C) on réduit ensuite en milieu aqueux, et

D) suivant le cas désiré, on traite à chaud avant la stérilisation, on met en forme et/ou on "soude" les pièces.

**Claims**

1. Collagen preparations out of collagen-I for the human or veterinary medicine obtainable by proteolyzing collagen-I material from mammals while preserving the biological texture thereof, cross-linking with dialdehydes and sterilizing, characterized in that one

A) performs the proteolysis in presence of ficin and L-cysteine in an aqueous medium at a pH-value of 5.5—6.5 and at a temperature of 30 to 50°C,

B) performs the cross-linking with one or more aliphatic dialdehydes having 2 to 10 carbon atoms,

C) reduces then in an aqueous medium and

D) optionally treats with heat before the sterilizing, molds and/or "welds" pieces.

2. Collagen preparations according to claim 1 obtainable by using dialdehydes with 4 to 7 carbon atoms, in particular glutaric dialdehyde, preferably in a 1—10% percent aqueous solution, for the cross-linking, performing the reduction in an aqueous medium with alkali metal borohydride and performing the heat treatment with hot air being saturated with steam at 80° to 100°C for some minutes and, if the preparations or pieces thereof shall be molded and/or if preparation pieces shall be "welded", coating an inert body of smooth surface having the desired shape with the preparations or preparation pieces the edges of which being closely adjacent to each other and continuing there the heat treatment for some minutes and then sterilizing.

3. Collagen preparations according to claim 1, obtainable thereby the one uses skin, corium, fasciae, tendines, blood vessels, tubular hollow organs, pericardia, and interstitial mammal tissue as mammalian colagen-I material and that one uses collagen-I material which is derived from goats, bovines and bovine fetusses.

4. Collagen preparations according to anyone of claims 1—3 obtainable by subjecting thick collagen material to a first proteolysis, then getting the desired thickness by cleaving it in the direction of the fibers, proteolyzing again the so thus obtained and continuing the treatment according to claim 1 or 2.

5. Collagen preparations according to anyone of claims 1, 2 or 4 obtainable by adding a treatment with diluted aqueous sodium chlorite solution after the proteolysis and optionally watering with running water between the individual process steps.

6. Collagen preparations according to anyone of claims 1 to 5 obtainable by treating mammalian collagen-I material with an aqueous solution containing 10 g ficin per liter and 1 g L-cysteine per liter at a pH value of 6 and at a temperature of about 37°C for 8 to 24 hours, then treating it with a 1% by weight aqueous sodium chlorite solution for 24 hours, watering it, treating it with a 1 to 10% aqueous glutaric dialdehyde solution for 7 days, watering it, treating it with an aqueous sodium borohydride solution for 24 hours, watering it and then sterilizing it, whereby the watering is performed with running water each for 24 hours.

7. Collagen preparations according to anyone of claims 1—6 obtainable thereby that, in order to obtain tubular hollow organs having a defined diameter, one coats a bar shaped, solid body having a smooth surface and the desired diameter, preferably a glass rod coated with silicone, with an adequately shaped collagen material after reduction and after the first short heat treatment, treats it with hot air being saturated with steam at about 1.5 bar at about 80 to 100°C for some minutes, and sterilizes it.

8. Collagen preparations according to anyone of claims 1—6 obtainable thereby that, in order to prepare a bag like hollow organ having a defined diameter, one coats a silicone coated glass flask of the desired diameter with a bovine pericardium after reduction and after the first heat treatment, continues the heat treatment for a short time, removes the shaped pericardium and sterilizes it.

9. Collagen preparations according to anyone of claims 1—6 obtainable thereby that, in order to prepare cotton like or woolly material, one uses rasped corium material or interstitial mamma tissue.

10. Collagen preparations according to any one of Claims 1, 2, 4, 6, 7 or 12, obtainable thereby that, in order to sterilize, one soaks the finished collagen preparation for some days in a 50% by weight aqueous ethyl alcohol containing about 1% by weight propylene oxide, transfers the preparation then into a sterile buffered sodium chloride solution, and heats the sodium chloride solution to evaporate the remaining sterilization solution during several hours.

11. Use of the collagen preparations according to anyone of claims 1—10 in the human veterinary medicine for surgical care of defective parts of the body, as transplant or implant, for substitution of skin, ligaments, tendons, tendon sheaths, hollow organs, blood vessels and the mamma.

12. Process for preparing the collagen preparations according to anyone of claims 1—10 wherein mammalian collagen-I material is proteolized by preserving the biological texture thereof, is cross-linked with dialdehydes and is sterilized, characterized in that

A) performs the proteolysis in presence of ficin and L-cysteine in an aqueous medium at a pH-value of 5.5—6.5 and at a temperature of 30 to 50°C.

B) performs the crosslinking with one or more aliphatic dialdehydes having 2 to 10 carbon atoms,

C) reduces then in an aqueous medium and

D) optionally treats with heat before the sterilyzing, molds and/or "welds" pieces.